**Europäisches Patentamt**

**European Patent Office**

**Office européen des brevets**

(19)

(11) Veröffentlichungsnummer: **0 347 714**

**A2**

(12) # EUROPÄISCHE PATENTANMELDUNG

(21) Anmeldenummer: **89110650.2**

(22) Anmeldetag: **13.06.89**

(51) Int. Cl.⁴: **A61K 37/18 , A61M 1/28**

(30) Priorität: **22.06.88 DE 3821043**

(43) Veröffentlichungstag der Anmeldung:
**27.12.89 Patentblatt 89/52**

(84) Benannte Vertragsstaaten:
**AT BE CH DE ES FR GB IT LI NL SE**

(71) Anmelder: **Fresenius AG**
**Gluckensteinweg 5**
**D-6380 Bad Homburg v.d.H.(DE)**

(72) Erfinder: **Steudle, Volker, Dr.**
**Hochelheimerweg 9**
**D-6300 Giessen(DE)**
Erfinder: **Bartz, Volker**
**Goethestrasse 44**
**D-6307 Linden(DE)**

(74) Vertreter: **Görtz, Dr. Fuchs, Dr. Luderschmidt**
**Patentanwälte**
**Abraham-Lincoln-Strasse 7 Postfach 46 60**
**D-6200 Wiesbaden(DE)**

(54) **Dialysier- und Spül-Lösung zur intraperitonealen Verabreichung.**

(57) Dialysier- und Spül-Lösungen zur intraperitonealen Verabreichung mit üblicher Elektrolytzusammensetzung in physiologischen Mengen und einem Aminosäuregemisch als osmotisch wirksame Substanz sowie gegebenenfalls zusätzlich organische Säure bzw. Salze derselben und gegebenenfalls weiteren Zusatzstoffen.

EP 0 347 714 A2

## Dialysier- und Spül-Lösung zur intraperitonealen Verabreichung

Gegenstand der vorliegenden Erfindung sind wäßrige Dialysier- und Spül-Lösungen zur intraperitonealen Verabreichung, enthaltend Elektrolyte in physiologischen Mengen, ein Aminosäuregemisch als osmotisch wirkende Substanz sowie gegebenenfalls weitere Zusatzstoffe.

Bei Patienten mit akuter oder chronischer Niereninsuffizienz muß die eingeschränkte Nierenfunktion durch alternative Verfahren kompensiert werden. Derartige alternative Verfahren sind die Hämodialyse und die Peritonealdialyse. Bei der Hämodialyse wird das Blut der Patienten mit Hilfe einer künstlichen semipermeablen Membran extrakorporal gereinigt. Bei der Peritonealdialyse dient das Peritoneum als semipermeable Membran. Die Dialysier-Lösung wird über einen Katheter in die Peritonealhöhle eingeführt. Nach einer bestimmten Equilibrierungszeit, das heißt nach konzentrationsabhängigem Austausch von Stoffen des Dialysats mit dem Blut, wird dann die "verbrauchte" Dialysier-Lösung aus der Peritonealhöhle entfernt und durch eine neue Dialysier-Lösung ersetzt. Die Peritonealdialyse, zum Beispiel die kontinuierliche ambulante Peritonealdialyse (CAPD) oder die intermittierende Peritonealdialyse gewinnt dabei zunehmend an Bedeutung. Während der Großteil der durch Urämie bedingten Manifestation bei einer Langzeit-Peritonealdialyse gut beherrscht werden kann, gibt es eine Anzahl metabolischer Defekte, die mit der verwendeten osmotisch wirksamen Substanz oder dem Puffer des Dialysats in Zusammenhang gebracht werden können.

Die wesentliche Aufgabe einer Dialysier-Lösung für niereninsuffiziente Patienten besteht einerseits darin, überschüssiges Wasser und harnpflichtige Substanzen aufzunehmen, und andererseits darin, Substanzen, die metabolisch bedingt in zu geringer Konzentration im Organismus vorkommen (Elektrolytbilanz), zuzuführen. Darüber hinaus soll durch eine Peritonealdialysier-Lösung für den Patienten ein positiver nutritiver Effekt erzielt werden, was insbesondere im Hinblick darauf von Bedeutung ist, als der CAPD-Patient täglich in das Dialysat etwa 5 bis 12 g Protein und einige g Aminosäuren abgibt.

Am verbreitetsten wurde bisher Glukose als osmotisch wirksame Substanz eingesetzt. So enthalten alle kommerziell verfügbaren Peritonealdialysier-Lösungen Glukose als osmotisch wirksame Substanz, um eine entsprechende Ultrafiltration zu gewährleisten. Glukose zeigt jedoch einige Nachteile. CAPD-Patienten absorbieren pro Tag 150 bis 300 g Glukose vom Dialysat. Diese Menge entspricht ungefähr einem Drittel des täglichen Kalorienbedarfs und trägt zur Obesitas bei. Insbesondere bei diabetischen Patienten werden Hyperglykämien mit einem erhöhten Insulinbedarf beschrieben. Dies zeigt sich besonders bei der intermittierenden Peritonealdialyse. Hyperglykämien führen des weiteren zu Hyperlipidämien, wodurch als Folge atherosklerotische Veränderungen hervorgerufen werden können. Aus der Peritonealhöhle wird Glukose rasch resorbiert, wodurch sich der osmotische Gradient zwischen Peritonealdialyse-Lösung und Plasma vermindert. Dies ist wiederum mit einem Rückgang der Ultrafiltration assoziiert. Ein weiterer Nachteil von Glukose ist der, daß sie ein hervorragendes Substrat für viele Mikroorganismen darstellt und daß dadurch die Entwicklung einer mikrobiellen Peritonitis begünstigt wird.

Um diese Nachteile und Probleme, die mit der Verwendung von Glukose als osmotisch wirksame Substanz in Dialysier-Lösungen verbunden sind, auszuschalten, wurden zahlreiche Untersuchungen hinsichtlich alternativer osmotisch wirksamer Substanzen unternommen. So wurde bereits seit längerem vorgeschlagen, Aminosäurelösungen anstelle von Glukose als osmotisch wirksame Substanzen einzusetzen (vergl. Lancet,Vol. 2, 1968, Seite 812).

Gemäß der PCT-Patentanmeldung WO 82/03773 wird eine Dialysier-Lösung vorgeschlagen, die eine wäßrige Lösung physiologischer Salze in zur osmotischen Verträglichkeit mit dem Blut ausreichender Konzentration, ein Gemisch physiologischer Aminosäuren und Insulin in ausreichender Menge enthalten, um für den Patienten eine wesentliche Assimilation der Aminosäuren zu gestatten. Als Beispiel für ein geeignetes Aminosäuregemisch ist in der PCT-Patentanmeldung das Handelsprodukt Travasol, das in der Dialysier-Lösung in Mengen von 1 bis 4 g pro l enthalten sein kann, genannt. Vorzugsweise enthalten die Dialysier-Lösungen gemäß dieser Patentanmeldung noch Glukose in Mengen von 0,5 bis 4 g pro l. Durch den Zusatz von Insulin zur Dialysier-Lösung soll sowohl die Metabolisierung der Glukose als auch der Aminosäuren sowie die Assimilation der Aminosäuren in die Zellen erleichtert werden.

Zwar werden durch diese Dialysier-Lösungen die Probleme der Hyperglykämie gemildert bzw. umgangen und geeignete osmotische Effekte erreicht, jedoch ist es mit diesen bekannten aminosäurehaltigen Dialysier-Lösungen nicht möglich, einen ausreichenden nutritiven Effekt bei den niereninsuffizienten Patienten zu erreichen.

Es besteht daher nach wie vor ein erheblicher Bedarf nach osmotisch wirksamen Dialysier-Lösungen, die über längere Zeit an Patienten verabreicht werden können, ohne daß auf osmotische Erscheinungen zurückzuführende Komplikationen oder mikrobielle Komplikationen auftreten, die einen ausreichenden Entzug von Wasser und harnpflichtigen Substanzen, eine Korrektur der Elektrolytbilanz und einen wirksamen nutritiven Effekt bei den niereninsuffizienten Patienten gewährleisten.

Aufgabe der vorliegenden Erfindung ist es daher, osmotisch wirksame Dialysier- und Spül-Lösungen zur Verfügung zu stellen, die über längere Zeit an Patienten intraperitoneal verabfolgt werden können, ohne daß auf osmotische Er scheinungen zurückzuführende Komplikationen auftreten, wobei die bei bisher bekannten Dialysier- und Spül-Lösungen auftretenden mikrobiellen und peritoneal dialytischen Komplikationen vermieden werden, ein ausreichender Entzug von Wasser und harnpflichtigen Substanzen und eine Korrektur der Elektrolytbilanz gewährleistet werden sowie für den Patienten durch die Dialysier-Lösung ein wirksamer nutritiver Beitrag sichergestellt wird.

Erfindungsgemäß wurde diese Aufgabe durch eine Dialysier-und Spül-Lösung gelöst, die als osmotisch wirksame Substanz ein Aminosäuregemisch aufweist, das die folgenden Aminosäuren in den folgenden relativen Mengen, ausgedrückt in Gew.-Teilen, enthält:

| L-Histidin | 4 | bis | 6 | Gew.-Teile |
|---|---|---|---|---|
| L-Isoleucin | 6 | bis | 9 | Gew.-Teile |
| L-Leucin | 9 | bis | 13,5 | Gew.-Teile |
| L-Methionin | 9 | bis | 12 | Gew.-Teile |
| L-Valin | 13,5 | bis | 20,5 | Gew.-Teile |
| L-Lysin-hydrochlorid | 6,5 | bis | 10 | Gew.-Teile |
| L-Phenylalanin | 6 | bis | 9,5 | Gew.-Teile |
| L-Threonin | 6,5 | bis | 10 | Gew.-Teile |
| L-Tyrosin | 7,5 | bis | 12 | Gew.-Teile |
| L-Taurin | 2 | bis | 8 | Gew.-Teile |
| L-Tryptophan | 1 | bis | 5 | Gew.-Teile. |

Vorzugsweise weist das erfindungsgemäß angewandte Aminosäuregemisch die folgenden Aminosäuren in den folgenden relativen Mengen, ausgedrückt in Gew.-Teilen, auf:

| L-Histidin | 4,5 | bis | 5 | Gew.-Teile |
|---|---|---|---|---|
| L-Isoleucin | 6 | bis | 6,5 | Gew.-Teile |
| L-Leucin | 9 | bis | 10 | Gew.-Teile |
| L-Methionin | 9 | bis | 10 | Gew.-Teile |
| L-Valin | 13,5 | bis | 15 | Gew.-Teile |
| L-Lysin-hydrochlorid | 6,5 | bis | 8 | Gew.-Teile |
| L-Phenylalanin | 6 | bis | 6,8 | Gew.-Teile |
| L-Threonin | 6,5 | bis | 7 | Gew.-Teile |
| L-Tyrosin | 7,5 | bis | 8,5 | Gew.-Teile |
| L-Taurin | 4,5 | bis | 6 | Gew.-Teile |
| L-Tryptophan | 2 | bis | 3 | Gew.-Teile. |

Insbesondere bevorzugt ist ein Aminosäuregemisch, das die Aminosäuren in den folgenden relativen Mengen enthält:

| L-Histidin | 4,9 | Gew.-Teile |
|---|---|---|
| L-Isoleucin | 6,0 | Gew.-Teile |
| L-Leucin | 9,0 | Gew.-Teile |
| L-Methionin | 9,0 | Gew.-Teile |
| L-Valin | 13,5 | Gew.-Teile |
| L-Lysin-hydrochlorid | 6,5 | Gew.-Teile |
| L-Phenylalanin | 6,0 | Gew.-Teile |
| L-Threonin | 6,5 | Gew.-Teile |
| L-Tyrosin | 7,5 | Gew.-Teile |
| L-Taurin | 4,9 | Gew.-Teile |
| L-Tryptophan | 2,5. | Gew.-Teile. |

Bei den erfindungsgemäß eingesetzten Aminosäuregemischen, ist es wesentlich, daß bestimmte Verhältnisse von Isoleucin : Leucin : Valin eingehalten werden. So sollte dieses Verhältnis derart sein, daß Isoleucin < Leucin < Valin ist. Vorzugsweise sollte das Verhältnis Isoleucin : Leucin : Valin 1 : 1,5 : 2,15 bis 2,25 betragen und beträgt insbesondere 1 : 1,5 : 2,25.

In gleicher Weise sollte das Verhältnis von Tyrosin : Phenylalanin > 1 sein. Vorzugsweise sollte es 1,05 bis 1,30 : 1 und insbesondere 1,25 : 1 betragen.

Das erfindungsgemäß angewandte Aminosäuregemisch wird in den erfindungsgemäßen Dialysier- und Spül-Lösungen in einer Menge von 2,0 bis 50.0 g Aminosäuregemisch pro l angewandt. Vorzugsweise enthalten die erfindungsgemäßen Dialysier- und Spül-Lösungen 7,5 bis 20 g Aminosäuren pro l und insbesondere 7,5 bis 12,5 g Aminosäuregemisch/l, beispielsweise 10 g Aminosäuregemisch pro l.

Erfindungsgemäß wurde überraschend gefunden, daß es unter Verwendung des vorstehend genannten Aminosäuregemisches möglich ist, bei der Behandlung der niereninsuffizienten Patienten einen wirksamen nutritiven Beitrag zu leisten. Die Zusammensetzung des erfindungsgemäß angewandten Aminosäuregemisches ist dem Bedürfnis der niereninsuffizienten Patienten angepaßt. Grundlage für die Zusammensetzung des Aminosäuregemisches bilden erfindungsgemäß nicht die Plasmaaminosäuremuster, sondern die Aminosäuremuster von intrazellulären relevanten Aminosäure-pools (Muskel).

Gemäß einer bevorzugten Ausführungsform enthalten die erfindungsgemäßen Dialysier- und Spül-Lösungen eine oder mehrere Carbonsäuren in Form ihrer Salze, zum Beispiel der Natrium-, Kalium- oder Calcium-Salze. Beispiele für geeignete Salze sind das Succinat, Fumarat, Malat, Oxalacetat und dergl. Bevorzugt verwendet wird Malat.

Diese Säuren bzw. deren Salze sind in den erfindungsgemäßen Lösungen in Mengen von 2 bis 12 g/l, vorzugsweise in einer Menge von 6,53 g/l vorhanden.

Beispiele für Elektrolytzusätze für die erfindungsgemäßen Dialysier- und Spül-Lösungen sind Natrium-, Kalium-, Calcium- oder Magnesiumionen enthaltende Elektrolyte. Die Elektrolytsalze können erfindungsgemäß in bekannter Weise in Form des Acetats, Lactats, Chlorids und/oder Bicarbonats vorliegen. Die Ionenkonzentrationen in den erfindungsgemäßen Dialysier- und Spül-Lösungen betragen vorteilhafterweise 125 bis 150, insbesondere 132 bis 140 mmol/l $Na^+$; 0 bis 8, insbesondee 0 bis 4 mmol/l $K^+$; 0 bis 3, insbesondere 0,5 bis 2 mmol/l $Ca^{++}$; 0 bis 2,5, insbesondere 0,3 bis 1 mmol/l $Mg^{++}$; 10 bis 60, insbesondere 30 bis 50 mmol/l Ionen, ausgewählt aus der Gruppe der Lactat-, Acetat- und Bicarbonationen und Rest $Cl^-$.

Die erfindungsgemäßen Dialysier- und Spül-Lösungen können ferner weitere geeignete physiologisch verträgliche Zusätze enthalten, die in Form von Lösungen oder Emulsionen den erfindungsgemäßen Lösungen zugesetzt werden. Beispiele für solche Zusätze sind Vitamine, wasserlösliche Kohlenhydrate, den Proteinstoffwechsel beeinflussende Hormone, Fettsäuren und/oder Fette, Glycerin, Glyceride sowie gegebenenfalls geeignete Emulgatoren. Diese Zusätze können erfindungsgemäß in solchen Mengen angewandt werden, wie sie in der Pharmakologie allgemein üblich sind.

Beispiele für Vitamine sind wasserlösliche und fettlösliche Vitamine, wie die Vitamine A, D, E, $B_1$, $B_2$, $B_6$ und $B_{12}$, Vitamin K, Vitamin C, Niacin, Pantothensäure, Biotin und Folsäure. Bevorzugt angewandt werden die Vitamine der Vitamin B-Gruppe, beispielsweise Vitamin $B_1$, $B_2$ und $B_6$, Pantothensäure, Niacin, Biotin und Vitamin C. Die Vitamine können einzeln oder im Gemisch eingesetzt werden. Die erfindungsgemäßen Dialysier- und Spül-Lösungen enthalten die Vitamine gewöhnlich in einer Menge von 10 bis 500 mg/l, vorzugsweise in einer Menge von 100 bis 200 mg/l, beispielsweise in einer Menge von 150 mg/l.

Beispiele für wasserlösliche Kohlenhydrate sind Monosaccharide, wie Glukose, Fructose, Galactose und Zuckeralkohole, wie Sorbit oder Xylit. Die Kohlenhydrate können einzeln oder im Gemisch verwendet werden. Vorzugsweise wird Glukose angewandt. Die erfindungsgemäßen Lösungen können die Kohlenhydrate in einer Menge von 0 bis 10 Gew.-%, vorzugsweise in einer Menge von 0 bis 5 Gew.-%, beispielsweise 1 Gew.-% enthalten.

Beispiele für den Proteinstoffwechsel beeinflussende Hormone sind Androstanolon oder Nortestosteron. Die Hormone können einzeln oder im Gemisch angewandt werden. In den erfindungsgemäßen Gemischen können diese Hormone im allgemeinen in einer pharmakologisch wirksamen Menge enthalten sein.

Beispiele für geeignete Fettsäuren sind Fettsäuren mit 5 bis 24 Kohlenstoffatomen. Beispiele für geeignete Fette sind ungesättigte Fette wie Sojabohnenöl, Baumwollsamenöl oder Fischöle.

Erfindungsgemäß werden im allgemeinen die Fette in Form von Emulsionen unter Verwendung eines geeigneten Emulgators, wie Lecitine, z. B. Soja-Lecitin oder gereinigtes Lecitin aus Hühnerei eingesetzt. Die Fette und/oder Fettsäuren sowie Glycerin oder Glyceride können in den erfindungsgemäßen Lösungen in Mengen von 0 bis 300 g/l, vorzugsweise 0 bis 200 g/l angewandt werden.

Der pH-Wert der erfindungsgemäßen Lösungen liegt im sauren Bereich, vorzugsweise im Bereich von 5,5 bis 6,5 und beträgt beispielsweise 5,6.

Der osmotische Druck der erfindungsgemäßen Dialysier- und Spül-Lösungen beträgt geeigneterweise 300 bis 700 mosm/l, vorzugsweise 320 bis 550 mosm/l und insbesondere 350 bis 500 mosm/l.

Die Herstellung der erfindungsgemäßen Dialysier- und Spül-Lösungen kann nach den zur Herstellung von Dialysier- und Spül-Lösungen bekannten Verfahren erfolgen. Beispielsweise kann die Herstellung derart erfolgen, wie sie in der PCT-Patentanmeldung WO 82/03773 beschrieben ist. Beispielsweise kann im Falle der zusätzlichen Verwendung von Kohlenhydraten die Sterilisierung in einem Doppelkammerbeutel erfolgen, wobei sich in einer Kammer die Kohlenhydratlösung (z. B. Glukoselösung) und in der anderen Kammer die Aminosäurelösung mit den weiteren Bestandteilen befindet, und kann nach der Sterilisierung unter Aufrechterhaltung der sterilen Bedingungen eine Verbindung der Kammern untereinander hergestellt werden und können so die beiden separat sterilisierten Lösungen vermischt werden.

Die erfindungsgemäßen Dialysier- und Spül-Lösungen sind ausgezeichnet zur peritonealen Verabreichung geeignet. Sie sind in hohem Maße wirksam (ausreichender Entzug von Wasser und harnpflichtigen Substanzen und geeignete Korrektur der Elektrolytbilanz) und können über längere Zeiträume verabreicht werden, ohne daß die bisher für die Dialysier-Lösungen bekannten mikrobiellen und peritonealdialytischen Komplikationen auftreten. Durch die erfindungsgemäßen Dialysier- und Spül-Lösungen werden Hyperglykämien, Obesitas, Appetitmangel, Hyperlipidämie, besonders bei diabetischen Patienten, vermieden. Das Ultrafiltrationsmaximum wird durch die erfindungsgemäßen Lösungen wesentlich früher als bei den bekannten, Glukose als osmotisch wirksame Substanz enthaltenden Dialysier-Lösungen erreicht, wodurch sie besonders gut zur schnellen Entwässerung geeignet sind. Durch die erfindungsgemäßen Lösungen wird darüber hinaus ein wirksamer nutritiver Beitrag für den Niereninsuffizienten sichergestellt. Der tägliche Aminosäureverlust wird durch diese Mittel korrigiert und die Proteinsynthese damit stimuliert, wodurch eine allgemeine Verbesserung des Patientenstatus gewährleistet wird.

Die nachfolgenden Beispiele dienen der weiteren Erläuterung der vorliegenden Erfindung:

Beispiel 1:

a) In einem Liter Wasser von Injektionsqualität wird eine Lösung folgender Aminosäuren hergestellt:

| | |
|---|---|
| L-Histidin | 4,9 g/l |
| L-Isoleucin | 6,0 g/l |
| L-Leucin | 9,0 g/l |
| L-Methionin | 9,0 g/l |
| L-Valin | 13,5 g/l |
| L-Lysin-hydrochlorid | 6,5 g/l |
| L-Phenylalanin | 6,0 g/l |
| L-Threonin | 6,5 g/l |
| L-Tyrosin | 7,5 g/l |
| L-Taurin | 4,9 g/l |
| L-Tryptophan | 2,5 g/l. |

Diese Lösung wurde auf eine Endkonzentration von 10 g/l Aminosäuren verdünnt.

b) Unter Verwendung der vorstehend beschriebenen Lösung wurde eine erfindungsgemäße Dialysier- und Spül-Lösung hergestellt. Diese Lösung enthielt in einem l Wasser von Injektionsqualität die folgenden Bestandteile:

| Aminosäuregemisch gemäß a) | 10 | g/l |
|---|---|---|
| L-Apfelsäure | 6,53 | g/l |
| NaCL | 5,785 | g/l |
| CaCl$_2$x2H$_2$O | 0,2573 | g/l |
| MgCl$_2$x6H$_2$O | 0,1017 | g/l |
| Na-lactat, 50 %ige Lösung | 10,76 | g/l (48 mmol/l) |
| Glukose | 10,0 | g/l |
| Pyridoxin-HCL | 40,0 | mg/l |
| Riboflavin-5-phosphat | 2,5 | mg/l |
| Nicotinamid | 60 | mg/l |
| Thiamin | 10 | mg/l |

Der theoretische osmotische Druck dieser so hergestellten Lösung betrug 500 mosm/l; pH-Wert: 5,6.

Beispiel 2:

Beispiel 1 wurde wiederholt mit der Ausnahme, daß das Aminosäuregemisch gemäß a) von Beispiel 1 in einer Menge von 7,5 g/l eingesetzt wurde.

Beispiel 3:

Das Beispiel 1 wurde wiederholt mit der Ausnahme, daß das Aminosäuregemisch gemäß a) von Beispiel 1 in einer Menge von 12,5 g/l eingesetzt wurde.

Beispiel 4:

Eine erfindungsgemäße Dialysier- und Spül-Lösung wurde aus den nachfolgend angegebenen Bestandteilen hergestellt:

| Na$^+$ | 134,0 | mmol/l |
| Ca$^{++}$ | 1,75 | mmol/l |
| Mg$^{++}$ | 0,50 | mmol/l |
| Cl$^-$ | entsprechend | |
| Lactat | 48 | mmol/l |
| L-Histidon | 0,6 | g/l |
| L-Isoleucin | 0,8 | g/l |
| L-Leucin | 1,2 | g/l |
| L-Methionin | 1,2 | g/l |
| L-Valin | 1,80 | g/l |
| L-Lysin-hydrochlorid | 0,85 | g/l |
| L-Phenylalanin | 0,8 | g/l |
| L-Threonin | 0,85 | g/l |
| L-Tyrosin | 1,0 | g/l |
| L-Taurin | 0,65 | g/l |
| L-Tryptophan | 0,3 | g/l |
| L-Apfelsäure | 6,5 | g/l |
| Pyridoxin | 40 | mg/l |
| Riboflavin-5-phosphat | 2,5 | mg/l |
| Nicotinamid | 60 | mg/l |
| Thiamin | 10 | mg/l |
| Pantothensäure | 10 | mg/l |
| Ascorbinsäure | 35 | mg/l |
| Biotin | 0,1 | mg/l |

Der theoretische osmotische Druck der Lösung betrug 500 mosm/l; pH-Wert: 5,6.

**Ansprüche**

1. Wäßrige Dialysier- und Spül-Lösung zur intraperitonealen Verabreichung, enthaltend Elektrolyte in physiologischen Mengen, ein Aminosäuregemisch als osmotisch wirksame Substanz und gegebenenfalls weitere Zusatzstoffe, **dadurch gekennzeichnet**, daß das Aminosäuregemisch die folgenden Aminosäuren in den folgenden relativen Mengen, ausgedrückt in Gewichtsteilen, enthält:

| L-Histidin | 4 | bis | 6 | Gew.-Teile |
| L-Isoleucin | 6 | bis | 9 | Gew.-Teile |
| L-Leucin | 9 | bis | 13,5 | Gew.-Teile |
| L-Methionin | 9 | bis | 12 | Gew.-Teile |
| L-Valin | 13,5 | bis | 20,5 | Gew.-Teile |
| L-Lysin-hydrochlorid | 6,5 | bis | 10 | Gew.-Teile |
| L-Phenylalanin | 6 | bis | 9,5 | Gew.-Teile |
| L-Threonin | 6,5 | bis | 10 | Gew.-Teile |
| L-Tyrosin | 7,5 | bis | 12 | Gew.-Teile |
| L-Taurin | 2 | bis | 8 | Gew.-Teile |
| L-Tryptophan | 1 | bis | 5 | Gew.-Teile |

2. Lösung nach Patentanspruch 1, **dadurch gekennzeichnet**, daß das Aminosäuregemisch die folgende Zusammensetzung, ausgedrückt in Gew.-Teilen, aufweist:

| L-Histidin | 4,5 | bis | 5 | Gew.-Teile |
|---|---|---|---|---|
| L-Isoleucin | 6 | bis | 6,5 | Gew.-Teile |
| L-Leucin | 9 | bis | 10 | Gew.-Teile |
| L-Methionin | 9 | bis | 10 | Gew.-Teile |
| L-Valin | 13,5 | bis | 15 | Gew.-Teile |
| L-Lysin-hydrochlorid | 6,5 | bis | 8 | Gew.-Teile |
| L-Phenylalanin | 6 | bis | 6,8 | Gew.-Teile |
| L-Threonin | 6,5 | bis | 7 | Gew.-Teile |
| L-Tyrosin | 7,5 | bis | 8,5 | Gew.-Teile |
| L-Taurin | 4,5 | bis | 6 | Gew.-Teile |
| L-Tryptophan | 2 | bis | 3 | Gew.-Teile. |

3. Lösung nach Patentanspruch 1 oder 2, **dadurch gekennzeichnet,** daß das Verhältnis der Aminosäuren Isoleucin : Leucin : Valin 1 : 1,5 : 2,25 beträgt.

4. Lösung nach Patentanspruch 1 bis 3, **dadurch gekennzeichnet,** daß das Verhältnis der Aminosäuren Tyrosin : Phenylalanin 1,25 : 1 beträgt.

5. Lösung nach Patentanspruch 1 bis 4, **dadurch gekennzeichnet,** daß sie das Aminosäuregemisch in einer Menge entsprechend 2 bis 50 g Aminosäuren/l enthält.

6. Lösung nach Patentanspruch 5, **dadurch gekennzeichnet,** daß sie die Aminosäuren in einer Menge entsprechend 7,5 bis 20 g Aminosäuren/l enthält.

7. Lösung nach Patentanspruch 6, **dadurch gekennzeichnet,** daß sie die Aminosäuren in einer Menge entsprechend 7,5 bis 12,5 g Aminosäuren/l enthält.

8. Lösung nach Patentanspruch 1 bis 7, **dadurch gekennzeichnet,** daß sie einen osmotischen Druck im Bereich von 300 bis 700 mosm/l aufweist.

9. Lösung nach Patentanspruch 8, **dadurch gekennzeichnet,** daß der osmotische Druck 320 bis 550 mosm/l beträgt.

10. Lösung nach Patentanspruch 1 bis 9, **dadurch gekennzeichnet,** daß der pH-Wert im sauren Bereich liegt.

11. Lösung nach Patentanspruch 10, **dadurch gekennzeichnet,** daß der pH-Wert 5,5 bis 6,5 beträgt.

12. Lösung nach Patentanspruch 1 bis 11, **dadurch gekennzeichnet,** daß sie die Elektrolyte in einer Konzentration von 125 bis 150 mmol/l $Na^+$, 0 bis 8 mmol/l $K^+$, 0 bis 3 mmol/l $Ca^{++}$, 0 bis 2,5 mmol/l $Mg^{++}$, 10 bis 60 mmol/l Ionen ausgewählt aus der Gruppe: Lactationen, Acetationen, Bicarbonationen und Rest $Cl^-$ enthält.

13. Lösung nach Patentanspruch 1 bis 12, **dadurch gekennzeichnet,** daß sie zusätzlich ein Carbonsäuresalz enthält.

14. Lösung nach Patentanspruch 13, **dadurch gekennzeichnet,** daß das Carbonsäuresalz ein Succinat, Fumarat, Malat oder Oxalacetat ist.

15. Lösung nach Patentanspruch 1 bis 14, **dadurch gekennzeichnet,** daß sie zusätzlich wasserlösliche Kohlenhydrate, Vitamine, den Proteinstoffwechsel beeinflussende Hormone und/oder Fette enthält.